(19) Europäisches Patentamt
European Patent Office
Office européen des brevets

(11) Veröffentlichungsnummer: **0 332 076 B1**

# (12) EUROPÄISCHE PATENTSCHRIFT

(45) Veröffentlichungstag der Patentschrift: **14.09.94**

(51) Int. Cl.[5]: **C07C 249/00**, A01N 35/10

(21) Anmeldenummer: **89103750.9**

(22) Anmeldetag: **03.03.89**

Die Akte enthält technische Angaben, die nach dem Eingang der Anmeldung eingereicht wurden und die nicht in dieser Patentschrift enthalten sind.

## (54) Cyclohexenonverbindungen, Verfahren zu ihrer Herstellung und ihre Verwendung zur Bekämpfung unerwünschten Pflanzenwuchses.

(30) Priorität: **11.03.88 DE 3808072**

(43) Veröffentlichungstag der Anmeldung:
**13.09.89 Patentblatt 89/37**

(45) Bekanntmachung des Hinweises auf die Patenterteilung:
**14.09.94 Patentblatt 94/37**

(84) Benannte Vertragsstaaten:
**BE CH DE FR GB IT LI NL**

(56) Entgegenhaltungen:
**EP-A- 0 054 682**
**EP-A- 0 137 174**
**GB-A- 2 124 198**
**US-A- 4 432 786**

**CHEMICAL ABSTRACTS, Band 102, Nr. 11, 18. März 1985, Seite 549, ZusammenfassungNr. 95395u, Columbus, Ohio, US; & JP-A-59 155 353**

(73) Patentinhaber: **BASF Aktiengesellschaft**
**Carl-Bosch-Strasse 38**
**D-67063 Ludwigshafen (DE)**

(72) Erfinder: **Kolassa, Dieter, Dr.**
**Moltkestrasse 8**
**D-6700 Ludwigshafen (DE)**
Erfinder: **Kast, Jürgen, Dr.**
**Kastanienstrasse 24**
**D-6737 Boehl-Iggelheim (DE)**
Erfinder: **Keil, Michael, Dr.**
**Fontanestrasse 4**
**D-6713 Freinheim (DE)**
Erfinder: **Schirmer, Ulrich, Dr.**
**Berghalde 79**
**D-6900 Heidelberg (DE)**
Erfinder: **Meyer, Norbert, Dr.**
**Dossenheimer Weg 22**
**D-6802 Ladenburg (DE)**
Erfinder: **Westphalen, Karl-Otto, Dr.**
**Mausbergweg 58**
**D-6720 Speyer (DE)**
Erfinder: **Würzer, Bruno, Dr.**
**Rüdigerstrasse 13**
**D-6701 Otterstadt (DE)**

EP 0 332 076 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Cyclohexenonverbindung der Formel I,

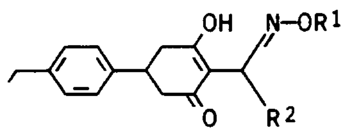

in der

$R^1$ eine $C_3$-$C_4$-Alkenylgruppe oder eine $c_2$-$C_4$-Halogenalkenylgruppe und

$R^2$ eine $C_1$-$C_4$-Alkylgruppe bedeuten,

sowie deren landwirtschaftlich annehmbaren Salze.

Außerdem betrifft die Erfindung ein verfahren zur Herstellung dieser Verbindungen sowie verfahren zur Bekämpfung unerwünschten Pflanzenwuchses, mit Mitteln, die die Verbindungen I enthalten.

Die Verbindungen I können in mehreren tautomeren und stereoisomeren (E- und Z-Isomerie) Formen auftreten, die alle vom Anspruch umfaßt werden.

In der Literatur werden 3-Hydroxy-2-cyclohexen-1-one beschrieben, die in 5-Position einen substituierten Phenylrest tragen und zur Bekämpfung von unerwünschten Gräsern in breitblättrigen Kulturen geeignet sind (DE-A 24 39 104). Desweiteren sind phenylsubstituierte Cyclohexenonverbindungen bekannt, welche zur Bekämpfung von Grasunkräutern in Kulturen wie Mais, Weizen, Gerste und Reis dienen (DE-A 32 48 554, DE-A 33 29 017, DE-A 30 47 924 und U.S. 4,432,786).

Es werden jedoch Verbindungen gesucht, die bei geringer Aufwandmenge hohe Selektivität aufweisen, d.h. unerwünschte Pflanzen bekämpfen, ohne dabei die Kulturpflanzen zu schädigen.

Entsprechend dieser Aufgabe wurden die eingangs definierten Cyclohexenonverbindungen gefunden.

Die Cyclohexenone der Formel I können auf bekannte Weise aus schon bekannten Rohstoffen erhalten werden; so kann man ein entsprechendes Cyclohexenon der Formel II

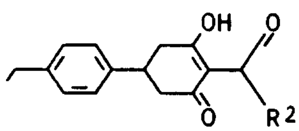

II,

mit einem Hydroxylamin $R^1ONH_2$ umsetzen.

Zweckmäßig führt man die Umsetzung in heterogener Phase in einem Lösungsmittel, bei Temperaturen bis 80 °C in Gegenwart einer Base durch und verwendet als Hydroxylaminquelle dessen Ammoniumverbindung.

Geeignete Basen sind z.B. Carbonate, Hydrogencarbonate, Acetate, Alkoholate oder Oxide von Alkali- oder Erdalkalimetallen, insbesondere Natriumhydroxid, Kaliumhydroxid, Magnesiumoxid, Calciumoxid. Außerdem können organische Basen wie Pyridin oder tertiäre Amine Verwendung finden. Die Base wird beispielsweise in einer Menge von 0,5 bis 2 Mol, bezogen auf die Ammoniumverbindung zugesetzt (DE-A-34 33 767).

Als Lösungsmittel eignen sich beispielsweise Dimethylsulfoxid, Alkohole wie Methanol, Ethanol und Isopropanol, aromatische Kohlenwasserstoffe wie Benzol und Toluol, chlorierte Kohlenwasserstoffe wie Chloroform und Dichlorethan, aliphatische Kohlenwasserstoffe wie Hexan und Cyclohexan, Ester wie Essigsäureethylester, Ether wie Dioxan und Tetrahydrofuran.

Die Umsetzung ist nach wenigen Stunden beendet, das Zielprodukt kann durch Einengen der Mischung, Verteilung des Rückstandes in Methylenchlorid/Wasser und Abdestillieren des Lösungsmittels unter vermindertem Druck isoliert werden.

Man kann auch unmittelbar die freie Hydroxylaminbase, z.B. in Form einer wäßrigen Lösung, verwenden; je nach dem verwendeten Lösungsmittel für den anderen Reaktionsteilnehmer erhält man ein ein- oder zweiphasiges Reaktionsgemisch.

Geeignete Lösungsmittel für diese Umsetzung sind beispielsweise Alkohole wie Methanol, Ethanol, Isopropanol und Cyclohexanol, aliphatische und aromatische, gegebenenfalls chlorierte Kohlenwasserstoffe, wie Hexan, Cyclohexan, Methylenchlorid, Toluol und Dichlorethan, Ester wie Essigsäureethylester, Nitrile

wie Acetonitril und cyclische Ether wie Tetrahydrofuran.

Alkalimetallsalze der Verbindungen 1 können durch Behandeln der 3-Hydroxyverbindungen mit Natrium- oder Kaliumhydroxid oder -alkoholat in wäßriger Lösung oder in einem organischen Lösungsmittel wie Methanol, Ethanol, Aceton, Toluol erhalten werden.

Andere Metallsalze, z.B. die Mangan-, Kupfer-, Zink-, Eisen-, Calcium-, Magnesium- und Bariumsalze können aus den Natriumsalzen in üblicher Weise hergestellt werden, ebenso Ammonium- und Phosphonium-, Sulfonium- und Sulfoxoniumsalze mittels Ammoniak, Phosphonium-, Sulfonium- oder Sulfoxoniumhydroxiden.

Die Verbindungen des Typs II können z.B. aus den entsprechenden Cyclohexan-1,3-dionen der Formel III

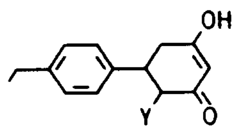

III,

wobei Y Wasserstoff oder Methoxycarbonyl bedeuten kann, mit bekannten Methoden [Tetrahedron Lett., 2491 (1975)] hergestellt werden.

Es ist auch möglich, die Verbindungen der Formel II über die Zwischenstufe der Enolester herzustellen, die bei der Umsetzung von Verbindungen der Formel III mit Säurechloriden in Gegenwart von Basen anfallen und anschließend mit bestimmten Imidazol- oder Pyridinderivaten umgelagert werden (JP-OS 79/063052).

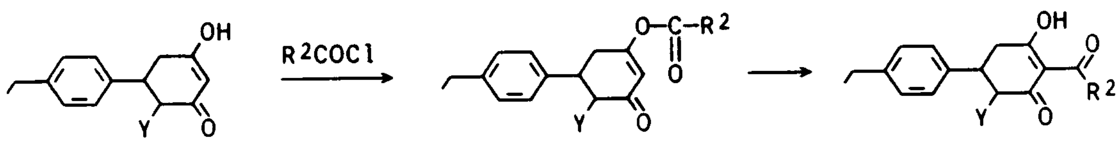


III

II

Zu Verbindungen der Formel I gelangt man, ausgehend von bekannten, handelsüblichen Verbindungen sowie durch eine Reihe bekannter Verfahrensschritte, wie dies aus folgendem Schema hervorgeht und durch ein praktisches Beispiel erläutert ist.

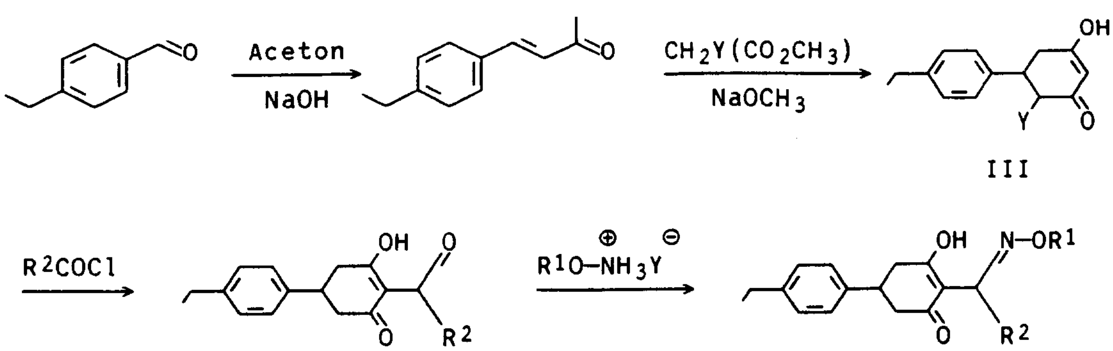


III

II

I

Im Hinblick auf die biologische Wirksamkeit sind Cyclohexenonverbindungen I bevorzugt, in denen die Reste folgende Bedeutung haben:

- $R^1$ eine Alkenylgruppe wie Allyl, Buten-2-yl, Buten-3-yl und 2-Methyl-buten-2-yl, insbesondere Allyl und Buten-2-yl;
  eine Halogenalkenylgruppe wie 2-Chlorprop-2-enyl, 3-Chlorprop-2-enyl, 3,3,2-Trichlorprop-2-enyl, insbesondere 3-Chlorprop-2-enyl;
- $R^2$ $C_1$-$C_4$-Alkyl wie Methyl, Ethyl, Propyl, 1-Methylethyl, Butyl, 1-Methylpropyl, 2-Methylpropyl und

1,1-Dimethylethyl, insbesondere Ethyl und Propyl.

Als Salze der Verbindungen der Formel 1 kommen landwirtschaftlich annehmbare Salze, beispielsweise Alkalimetallsalze, insbesondere Kalium- oder Natriumsalze, Erdalkalimetallsalze, insbesondere Calcium- und ferner Mangan-, Kupfer-, Zink- oder Eisensalze, Ammonium-, Tetraalkylammonium-, Benzyltrialkylammonium-, Trialkylsulfoxonium- oder Trialkylsulfoniumsalze in Betracht.

Die Cyclohexenone bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolethern, Kondensationsproduckte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Die Cyclohexenonverbindungen können beispielsweise wie folgt formuliert werden.

I. Man vermischt 90 Gewichtsteile der Verbindung Nr. 1 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 8 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 3 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 9 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 13 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 12 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,01 bis 3,0, vorzugsweise 0,05 bis 1,0 kg/ha.

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |

| Botanischer Name | Deutscher Name |
|---|---|
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |

| Botanischer Name | Deutscher Name |
|---|---|
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Cyclohexenone der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die neuen Verbindungen der Formel 1 allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Herstellungsbeispiele

Die in den nachstehenden Synthesebeispielen wiedergegebenen Vorschriften wurden unter entsprechender Abwandlung der Ausgangsverbindungen zur Gewinnung weiterer Verbindungen der Formel I benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

Beispiel 1

a) 4-(4-Ethylphenyl)-3-buten-2-on
Eine Mischung aus 111,4 g (0,83 mol) 4-Ethylbenzaldehyd, 133 g Aceton und 100 ml Wasser wird mit 20 ml 10 %iger Natronlauge versetzt und über Nacht bei Raumtemperatur gerührt. Dann wird mit 300 ml Eiswasser verdünnt und fünfmal mit je 100 ml Dichlormethan extrahiert. Man trocknet über Natriumsulfat und engt ein.
Ausbeute: 144 g eines gelben Öls, das wie folgt weiter umgesetzt wird:
b) 5-(4-Ethylphenyl)-3-hydroxy-2-cyclohexen-1-on
109 g (0,83 mol) Malonsäuredimethylester und 45 g (0,83 mol) Natriummethylat werden in 40 ml Methanol vorgelegt, 144 g (0,83 mol) 4-(4-Ethylphenyl)-3-buten-2-on, gelöst in wenig Methanol, zugetropft. Man rührt 3 h bei Rückfluß und 10 h bei Raumtemperatur. Das Methanol wird abdestilliert, der Rückstand in 2 l 10 %iger Kalilauge aufgenommen und 8 h bei Raumtemp. gerührt. Bei 60 °C wird mit konz. Salzsäure auf pH 1 angesäuert. Man rührt noch weitere 2 h, saugt ab und trocknet i.V.
Ausbeute: 167 g eines gelben Feststoffs Schmp. 162 - 64 °C
c) 2-Butyryl-5-(4-ethylphenyl)-3-hydroxy-2-cyclohexen-1-on
Zur Lösung von 110 g (0,51 mol) des nach der vorstehenden Vorschrift erhaltenen 5-(4-Ethylphenyl)-3-hydroxy-2-cyclohexen-1-ons in 500 ml Tetrahydrofuran werden 51 g (0,51 mol) Triethylamin und unter Eiskühlung 54 g (0,51 mol) Buttersäurechlorid getropft. Nach 8 h bei Raumtemperatur wird filtriert und mit Tetrahydrofuran gewaschen. Das Filtrat wird eingeengt, in 500 ml Essigsäureethylester aufgenommen und mit 6,2 g 4-N,N-Dimethylaminopyridin versetzt. Die homogene Lösung verbleibt 40 h bei Raumtemperatur, wird dann eingeengt und in 10 %iger Kalilauge aufgenommen. Man trennt vom Ungelösten ab und säuert das Filtrat auf pH 1 an. Das ausgefallene Produkt wird abgesaugt und i.V. getrocknet.

Ausbeute: 100 g eines gelben Feststoffs Schmp. 60 - 62 °C

d) 2-(E)-3-Chlor-2-propenyloxyiminobutyl-5-(4-ethylphenyl)-3-hydroxy-2-cyclohexen-1-on

Man legt 30 g (0,11 mol) des nach Vorschrift c) erhaltenen 2-Butyryl-5-(4-ethylphenyl)-3-hydroxy-2-cyclohexen-1-ons in 300 ml Methanol vor, gibt 16,6 g (0,12 mol) (E)-3-Chlor-2-propenyloxyaminhydrochlorid und 9,7 g (0,12 mol) Natriumhydrogencarbonat zu und rührt bei Raumtemperatur über Nacht. Man engt ein, nimmt in 300 ml Dichlormethan auf und wäscht zweimal mit Wasser. über Natriumsulfat trocknen und einengen.

Ausbeute: 22 g eines gelben Feststoffs (Wirkstoff Nr. 6 in der folgenden Tabelle)

Schmp. 56-58 °C

Die in der folgenden Tabelle genannten Verbindungen der Formel I sind durch ihren Schmelzpunkt (Schmp.) und/oder $^1$H-NMR-Daten charakterisiert.

Die $^1$H-NMR-Spektren wurden in Deuterochloroform oder Hexadeuterosulfoxid als Lösungsmittel mit Tetramethylsilan als innerem Standard aufgenommen. Die chemischen Verschiebungen werden in (ppm) registriert. Die Multiplizitäten werden wie folgt angegeben: s = Singulett, d = Dublett, t = Triplett, q = Quartett, m = Multiplett.

Tabelle

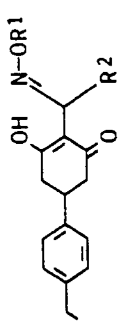

I,

| Wirkstoff Nr. | $R^2$ | $R^1$ | Fp. (°C) | $^1H$-NMR ($\delta$ in ppm) |
|---|---|---|---|---|
| 1 | Ethyl | $CH_2CH=CH_2$ | | 1.15(t, 3H), 1.22(t, 3H), 2.64(q, 2H), 4.54(d, 2H) |
| 2 | Ethyl | (E)-$CH_2CH=CHCH_3$ | 53-54 | 1.16(t, 3H), 1.23(t, 3H), 1.77(d, 3H), 2.64(q, 2H), 4.47(d, 2H) |
| 3 | Ethyl | (E)-$CH_2CH=CHCl$ | | 1.16(t, 3H), 1.23(t, 3H), 2.64(q, 2H), 4.52(d, 2H) |
| 4 | n-Propyl | $CH_2CH=CH_2$ | | 0.98(t, 3H), 1.24(t, 3H), 2.63(q, 2H) 4.54(d, 2H) |
| 5 | n-Propyl | (E)-$CH_2CH=CHCH_3$ | | 0.99(t, 3H), 1.25(t, 3H), 1.77(d, 3H), 2.64(q, 2H), 4.45(d, 2H) |
| 6 | n-Propyl | (E)-$CH_2CH=CHCl$ | 56-58 | 0.97(t, 3H), 1.24(t, 3H), 2.63(q, 2H), 4.52(d, 2H) |

Anwendungsbeispiele

Die Wirkung der Cyclohexenonderivate der Formel I auf das Pflanzenwachstum läßt sich durch Gewächshausversuche Zeigen:

Zur Anzucht der Pflanzen dienen Plastikblumentöpfe mit 300 $cm^3$ Inhalt und lehmigem Sand mit etwa 3 % Humus als Substrat. Die Samen der Testpflanzen werden nach Arten getrennt flach eingesät.

Bei Vorauflaufbehandlung werden die aufbereiteten Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewaschsen sind. Diese Abdeckung bewirkt ein gleichmäßiges Keimen der Testpflanzen, sofern dies nicht durch die Wirkstoffe beeinträchtigt wird. Die Aufwandmengen betragen 0,5 kg/ha aktive Substanz (a.S.).

Zum Zwecke der Nachauflaufbehandlung zieht man die Testpflanzen je nach Wuchsform erst bis zu einer Wuchshöhe von 3 bis 15 cm an und behandelt sie danach. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung betragen 0,125 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| lateinischer Name | deutscher Name |
|---|---|
| Avena fatua | Flughafer |
| Alopecurus myosuroides | Ackerfuchsschwanz |
| Digitaria sanguinalis | Blutfingerhirse |
| Echinochlora crus-galli | Hühnerhirse |
| Lolium multiflorum | Ital. Raygras |
| Medicago sativa | Luzerne |
| Triticum aestivum | Weizen |
| Zea mays | Mais |

Die Wirkstoffe 3 und 6 zeigen bei Vorauflaufanwendung von 0,5 kg a.S./ha starke herbizide Wirkung gegen grasartige Beispielpflanzen, Senf als breitblättrige Spezies bleibt unbeeinflußt.

Zur Bekämpfung grasartiger Vegetation eignen sich die Beispiele Nr. 2 und 3 bei Nachauflaufanwendung von 0,125 kg a.S./ha. Breitblättrige Kulturen, wie am Beispiele von Luzerne gezeigt, werden dabei nicht geschädigt. Die neuen Wirkstoffe haben selektive herbizide Wirkung.

Der Wirkstoff 3 und 6 kann im Nachauflaufverfahren zur Bekämpfung unerwünschter Grasarten in der Gramineenkultur Weizen eingesetzt werden. Die Nutzpflanzen erleiden allenfalls eine unwesentliche Schädigung.

## Patentansprüche

**1.** Cyclohexenonverbindung der Formel I,

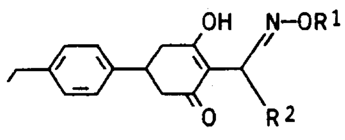

in der

$R^1$ eine $C_3$-$C_4$-Alkenylgruppe oder
eine $C_2$-$C_4$-Halogenalkenylgruppe und

$R^2$ eine $C_1$-$C_4$-Alkylgruppe bedeuten,

sowie deren landwirtschaftlich annehmbaren Salze.

**2.** Verfahren zur Herstellung von Cyclohexenonverbindungen der Formel I gemäß Anspruch 1, dadurch gekennzeichnet, daß man ein entsprechendes Keton der Formel II

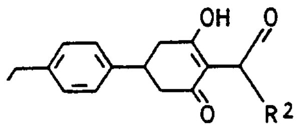

II,

mit einem entsprechend substituierten Hydroxylamin $R^1ONH_2$ umsetzt.

**3.** Herbizides Mittel, enthaltend ein Cyclohexenonderivat der Formel I gemäß Anspruch 1.

**4.** Herbizides Mittel, enthaltend übliche Zusatzstoffe und ein Cyclohexenonderivat der Formel I gemäß Anspruch 1 und gegebenenfalls weitere Wirkstoffe.

**5.** Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines Cyclohexenonderivats der Formel I gemäß Anspruch 1 behandelt.

**Claims**

**1.** A cyclohexenone compound of the formula I

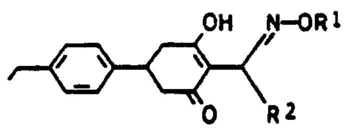

where $R^1$ is $C_3$- or $C_4$-alkenyl or $C_2$-$C_4$-haloalkenyl and $R^2$ is $C_1$-$C_4$-alkyl, and agriculturally acceptable salts thereof.

**2.** A process for the preparation of a cyclohexenone compound of the formula I as claimed in claim 1, wherein a corresponding ketone of the formula II

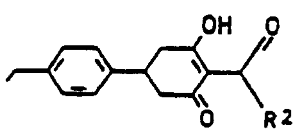

II,

is reacted with a correspondingly substituted hydroxylamine $R^1ONH_2$.

**3.** A herbicide containing a cyclohexenone derivative of the formula I as claimed in claim 1.

**4.** A herbicide containing conventional additives and a cyclohexenone derivative of the formula I as claimed in claim 1, and, if desired, further active ingredients.

**5.** A method for controlling undesirable plant growth, wherein the undesirable plants and/or their habitat are treated with a herbicidally effective amount of a cyclohexenone derivative of the formula I as claimed in claim 1.

## Revendications

**1.** Composé de cyclohexénone répondant à la formule I

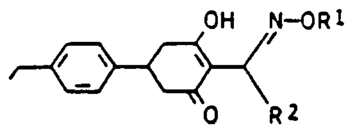

dans laquelle
$R^1$ représente un groupe alcényle en $C_3$-$C_4$ ou
un groupe halogénoalcényle en $C_2$-$C_4$ et
$R^2$ représente un groupe alkyle en $C_1$-$C_4$,
et leurs sels acceptables pour les usages agricoles.

**2.** Procédé de préparation des composés de cyclohexénone de formule I selon la revendication 1, caractérisé en ce que l'on fait réagir une cétone correspondante de formule II

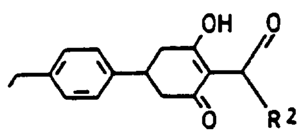

II,

avec une hydroxylamine portant le substituant approprié $R^1ONH_2$.

**3.** Produit herbicide contenant un composé de cyclohexénone de formule I selon la revendication 1.

**4.** Produit herbicide contenant des additifs usuels et un composé de cyclohexénone de formule I selon la revendication 1 avec le cas échéant d'autres substances actives.

**5.** Procédé pour combattre les croissances de végétaux indésirables, caractérisé en ce que l'on traite les végétaux indésirables et/ou leur habitat par une quantité herbicide efficace d'un composé de cyclohexénone de formule I selon la revendication 1.